# EUROPEAN PATENT SPECIFICATION

(11) **EP 3 358 941 B1**
(45) Date of publication and mention of the grant of the patent: **11.12.2024**
(21) Application number: 17701911.4
(22) Date of filing: 12.01.2017
(51) Int. Cl.: A01H 5/00, C12N 15/82

(54) **MODIFIED PLANT**
MODIFIZIERTE PFLANZE
PLANTE MODIFIÉE

(30) Priority: 13.01.2016 GB 201600631
(43) Date of publication of application: 15.08.2018
(73) Proprietor: Sun Pharmaceutical Industries Australia Limited, Notting Hill, Victoria 3168 (AU)
(72) Inventor: WINZER, Thilo, York Yorkshire YO10 5DD (GB); WALKER, Tracy Carol, Wilsonton Queensland 4350 (AU); MEADE, Fergus, Carlow (IE); LARSON, Tony Robert, York YO10 5DD (GB); GRAHAM, Ian Alexander, York Yorkshire YO10 5DD (GB)
(74) Representative: Symbiosis IP Limited
(86) International application number: PCT/GB2017/050068
(87) International publication number: WO 2017/122011

(56) References cited:
- WO-A1-2009/143574
- AU-A1- 2012 327 196
- S. C. FARROW ET AL: "Dioxygenases Catalyze O-Demethylation and O,O-Demethylenation with Widespread Roles in Benzylisoquinoline Alkaloid Metabolism in Opium Poppy", JOURNAL OF BIOLOGICAL CHEMISTRY, vol. 288, no. 40, 8 August 2013 (2013-08-08), US, pages 28997 - 29012, XP055361195, ISSN: 0021-9258, DOI: 10.1074/jbc.M113.488585
- CHAMPA P. WIJEKOON ET AL: "Systematic knockdown of morphine pathway enzymes in opium poppy using virus-induced gene silencing", THE PLANT JOURNAL, vol. 69, no. 6, 28 March 2012 (2012-03-28), pages 1052 - 1063, XP055067993, ISSN: 0960-7412, DOI: 10.1111/j.1365-313X.2011.04855.x
- AHMET GÃ 1/4 MÃ 1/4 AA Ã PRG Ã 1/4 ET AL: "Evaluation of selected poppy (Papaver somniferum L.) lines by their morphine and other alkaloids contents", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 226, no. 5, 2 September 2007 (2007-09-02), pages 1213 - 1220, XP019585076, ISSN: 1438-2385
- ELENA FOSSATI ET AL: "Synthesis of Morphinan Alkaloids in Saccharomyces cerevisiae", PLOS ONE, vol. 10, no. 4, 1 April 2015 (2015-04-01), pages e0124459, XP055317484, DOI: 10.1371/journal.pone.0124459
- JILLIAN M HAGEL ET AL: "Dioxygenases catalyze the O-demethylation steps of morphine biosynthesis in opium poppy", NATURE CHEMICAL BIOLOGY, vol. 6, no. 4, 14 March 2010 (2010-03-14), pages 273 - 275, XP055090844, ISSN: 1552-4450, DOI: 10.1038/nchembio.317
- "Plant Breeding from Laboratories to Fields", 22 May 2013, INTECH, ISBN: 978-953-51-1090-3, article BRIJ KISHORE ET AL: "Opium Poppy: Genetic Upgradation Through Intervention of Plant Breeding Techniques", XP055361815, DOI: 10.5772/53132

## Description

### Field of the invention

The disclosure relates to modified plants of the genus *Papaver* comprising one or more genes encoding a codeine 3-O-demethylase (CODM) wherein the modified plants have a modified alkaloid content when compared to unmodified plants wherein the alkaloid content has a high codeine phenotype; and including the use of nucleic acids encoding CODM and CODM variants for use as biomarkers in plant breeding.

### Background to the Invention

The genetic variation in a natural plant population offers a rich source of specific traits; however traditional plant breeding methods selecting a specific phenotype are slow and labour intensive. Mutagenesis of the germplasm through chemical mutagens or radiation creates a diverse range of modified alleles or deletion mutants with altered phenotype, often unobtainable when using traditional plant breeding techniques. Methods such as Targeting Induced Local Lesions in Genomes (TILLING), enable the detection of single base changes after chemical mutagenesis and has become an important tool for plant breeding. TILLING, in combination with marker assisted breeding, has resulted in the identification of new plant varieties with desired traits. TILLING is a high-throughput reverse genetic method to obtain allelic series from a chemically mutagenized population. A chosen target is amplified from pooled DNA using labelled PCR primers. Following amplification, the PCR products are denatured and re-annealed. If a mutation is present in the pooled DNA, a heteroduplex will form. A specific nuclease is then used to cleave a strand of the heteroduplex and the products are electrophoresed on a denaturing acrylamide gel. Mutations are detected by the observation of cleaved bands.

*Papaver somniferum,* the opium poppy, is a source of clinically useful opiate alkaloids such as morphine, codeine, thebaine, noscapine and papaverine. Opiate alkaloids have broad clinical application ranging from analgesics to cough suppressants and anti-spasmodics. Whilst opiate alkaloids can be extracted from latex harvested from the green seed pods of opium poppy or from the poppy straw which is the dried mature plant, the demand for the opiate alkaloids exceeds the available natural supply necessitating costly chemical synthesis. This particularly applies for codeine which is mostly obtained from morphine through a semisynthetic process as the levels of codeine found in plants account typically for less 5% of the level of morphine; however, codeine has been the most commonly consumed opiate in the world reaching 361 tons in 2013.

Codeine can be extracted directly from the plant and is also obtainable through a semisynthetic process by methylating morphine. In the poppy plant codeine and morphine are synthesised from (*R*)-reticuline through the intermediate thebaine involving several enzymatic steps. Thebaine is then converted to either oripavine by the codeine 3-O-demethylase (CODM) which is then subsequently transformed in several steps to morphine, or thebaine is transformed to codeine, which is, through the activity of the CODM, converted to morphine by de-methylation.

An increase of the codeine content in opium poppy has been reported after viral induced gene silencing targeting CODM expression. Similarly, in order to increase the codeine content in opium poppy, plants have been mutagenized and screened for high codeine as disclosed in US8541647. Mutagenesis resulted in *Papaver somniferum* plants with high codeine containing little or no oripavine, morphine or thebaine. The genotype of these plants however is unknown. Although chemical mutagenesis is an important tool to alter specific traits, it is a laborious and time intensive screening process. It would be desirable to identify gene markers that allow marker assisted selection of plants with a required phenotype.

The present disclosure relates to the deletion of one or more copies of the CODM resulting in plants with high codeine in the poppy straw and undetectable levels of morphine.

### Statement of the Invention

According to an aspect of the invention there is provided a *Papaver* plant species wherein the plant is modified by deletion for all or part of three linked genes encoding codeine 3-O-demethylase comprising the nucleotide sequence set forth in SEQ ID NO: 7, or a nucleotide sequence that is 99% identical to SEQ ID NO: 7, characterised in that the activity of said codeine 3-O-demethylase is undetectable and wherein the plant has a codeine content of at least 45% of the total alkaloid content of latex or dried straw extracted from the modified plant when compared to a wild-type *Papaver* plant of the same species.

In a preferred embodiment of the invention said reduced expression is the result of deletion of all or part of a codeine 3-O-demethylase gene encoded by a nucleic acid molecule comprising the nucleotide sequence set forth in SEQ ID NO: 1.

In a preferred embodiment of the invention said reduced expression is the result of deletion of all or part of a codeine 3-O-demethylase gene encoded by a nucleic acid molecule comprising the nucleotide sequence set forth in SEQ ID NO: 2.

In a preferred embodiment of the invention said reduced expression is the result of deletion of all or part of a codeine 3-O-demethylase gene encoded by a nucleic acid molecule comprising the nucleotide sequence set forth in SEQ ID NO: 3.

In a preferred embodiment of the invention said reduced expression is the result of deletion of all or part of a codeine 3-O-demethylase gene encoded by a nucleic acid molecule comprising the nucleotide sequence set forth in SEQ ID NO: 4.

In a preferred embodiment of the invention said reduced expression is the result of deletion of all or part of a codeine 3-O-demethylase gene encoded by a nucleic acid molecule comprising the nucleotide sequence set forth in SEQ ID NO: 5.

In a preferred embodiment of the invention said reduced expression is the result of deletion of all or part of a codeine 3-O-demethylase gene encoded by a nucleic acid molecule comprising the nucleotide sequence set forth in SEQ ID NO: 6.

In a preferred embodiment of the invention said reduced expression is the result of deletion of all or part of each codeine 3-O-demethylase gene encoded by a nucleic acid molecule comprising the nucleotide sequence set forth in SEQ ID NO: 1, 2, 3, 4, 5, 6 and 7 whereby said Papaver plant has no codeine 3-O-demethylase enzyme activity.

In a preferred embodiment of the invention said plant is modified by deletion of a nucleic acid molecule comprising or consisting of the nucleotide sequence as set forth in SEQ ID NO: 44.

Preferably, the plant is modified by deletion of a nucleic acid molecule comprising or consisting of a nucleotide sequence as set forth in SEQ ID NO: 44.

In a preferred embodiment of the invention said *Papaver* species is selected from the group consisting of: *Papaver somniferum, P. setigerum, P. orientale, P. pseudo-orientale, P. lasiothrix, P. cylindricum, P. fugax, P. triniifolium.*

In a preferred embodiment of the invention said *Papaver* species is *Papaver somniferum.*

"Total alkaloid content" is defined relative to the major alkaloids: morphine, codeine, noscapine, oripavine and thebaine. Dried straw refers to the dried stalks, stem and leaves of poppies minus the seeds.

In a preferred embodiment of the invention the codeine content is at least 50%, 55%, 60%, 65%, 70%, 80%, 85%, 90%, 95%, 96%, 97%, or 98% of the total alkaloid content of latex or dried straw extracted from said modified plant.

In an alternative embodiment of the invention the codeine content is at least 0.8% of the dried straw of said modified plant.

Preferably the codeine content is between, 0.8% and 10.0% of the dried straw; or between 0.8% and 3.0% of the dried straw or between 1.0 and 1.5% of the dried straw. Preferably the codeine content is about 1.1% of the dried straw.

Not covered by the claims there is provided an isolated nucleic acid molecule comprising a nucleotide sequence set forth in SEQ ID NO: 1, 2, 3, 4, 5, 6, 7, 8, 9, 10, 11, 12 or 13.

Preferably said isolated nucleic acid molecule comprises or consists of a nucleotide sequence as set forth in SEQ ID NO: 44, or a fragment thereof wherein said fragment comprises one or more codeine 3-O-demethylase genes.

Preferably said nucleic acid molecule is genomic DNA.

Preferablythe invention said nucleic acid molecule is cDNA.

Preferably said nucleic acid molecule comprises a nucleotide sequence wherein said sequence is degenerate as a result of the genetic code to the nucleotide sequence set forth in SEQ ID NO: 1, 2, 3, 4, 5, 6, 7, 8, 9, 10, 11, 12 or 13.

Preferably said nucleic acid molecule comprises or consists of a nucleotide sequence wherein said sequence is degenerate as a result of the genetic code to the nucleotide sequence as set forth in SEQ ID NO: 44.

Preferably said nucleic acid molecule encodes a codeine 3-O-demethylase polypeptide as represented in SEQ ID NO: 15, 16 or 17.

Not covered by the claims there is provided an expression vector comprising a nucleic acid molecule according to the invention adapted for expression in a microbial host cell.

Not covered by the claims there is provided a vector comprising a nucleic acid molecule or fragment thereof, comprising a nucleotide sequence as set forth in SEQ ID NO: 44, or a polymorphic sequence variant thereof, wherein said fragment comprises one or more codeine 3-O-demethylase genes

Not covered by the claims there is provided a microbial cell transformed with a nucleic acid molecule or vector according to the invention.

Preferably said microbial cell is a bacterial cell or fungal cell.

Not covered by the claims there is provided a microbial cell culture comprising a microbial cell according to the invention.

Not covered by the claims there is provided a fermenter comprising a microbial cell culture according to the invention.

Not covered by the claims there is provided a process for the modification of codeine comprising:
i) providing a transgenic microbial cell culture according to the invention that expresses a nucleic acid molecule encoding a codeine 3-O-demethylase in culture;
ii) cultivating the microbial cell under conditions that demethylates codeine to morphine; and optionally
iii) isolating the morphine from the microbial cell or cell culture.

If microbial cells are used as organisms in the process according to the invention they are grown or cultured in the manner with which the skilled worker is familiar, depending on the host organism. As a rule, microorganisms are grown in a liquid medium comprising a carbon source, usually in the form of sugars, a nitrogen source, usually in the form of organic nitrogen sources such as yeast extract or salts such as ammonium sulfate, trace elements such as salts of iron, manganese and magnesium and, if appropriate, vitamins, at temperatures of between 0°C and 100°C, preferably between 10°C and 60°C, while gassing in oxygen.

The pH of the liquid medium can either be kept constant, that is to say regulated during the culturing period, or not. The cultures can be grown batch wise, semi-batch wise or continuously. Nutrients can be provided at the beginning of the fermentation or fed in semi-continuously or continuously. Morphine produced can be isolated from the organisms as described above by processes known to the skilled worker, for example by extraction, distillation, crystallization, if appropriate precipitation with salt, and/or chromatography. To this end, the organisms can advantageously be disrupted beforehand. In this process, the pH value is advantageously kept between pH 4 and 12, preferably between pH 6 and 9, especially preferably between pH 7 and 8.

The culture medium to be used must suitably meet the requirements of the strains in question. Descriptions of culture media for various microorganisms can be found in the textbook "Manual of Methods for General Bacteriology" of the American Society for Bacteriology (Washington D.C., USA, 1981).

As described above, these media which can be employed in accordance with the invention usually comprise one or more carbon sources, nitrogen sources, inorganic salts, vitamins and/or trace elements.

Preferred carbon sources are sugars, such as mono-, di- or polysaccharides. Examples of carbon sources are glucose, fructose, mannose, galactose, ribose, sorbose, ribulose, lactose, maltose, sucrose, raffinose, starch or cellulose. Sugars can also be added to the media via complex compounds such as molasses or other by-products from sugar refining. The addition of mixtures of a variety of carbon sources may also be advantageous. Other possible carbon sources are oils and fats such as, for example, soya oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol, and/or organic acids such as, for example, acetic acid and/or lactic acid.

Nitrogen sources are usually organic or inorganic nitrogen compounds or materials comprising these compounds. Examples of nitrogen sources comprise ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as cornsteep liquor, soya meal, soya protein, yeast extract, meat extract and others. The nitrogen sources can be used individually or as a mixture.

Inorganic salt compounds which may be present in the media comprise the chloride, phosphorus and sulfate salts of calcium, magnesium, sodium, cobalt, molybdenum, potassium, manganese, zinc, copper and iron.

Inorganic sulfur-containing compounds such as, for example, sulfates, sulfites, dithionites, tetrathionates, thiosulfates, sulfides, or else organic sulfur compounds such as mercaptans and thiols may be used as sources of sulfur for the production of sulfur-containing fine chemicals, in particular of methionine.

Phosphoric acid, potassium dihydrogenphosphate or dipotassium hydrogenphosphate or the corresponding sodium-containing salts may be used as sources of phosphorus.

Chelating agents may be added to the medium in order to keep the metal ions in solution.

Particularly suitable chelating agents comprise dihydroxyphenols such as catechol or protocatechuate and organic acids such as citric acid.

The fermentation media used according to the invention for culturing microorganisms usually also comprise other growth factors such as vitamins or growth promoters, which include, for example, biotin, riboflavin, thiamine, folic acid, nicotinic acid, panthothenate, α-ketoglutarate and pyridoxine. Growth factors and salts are frequently derived from complex media components such as yeast extract, molasses, cornsteep liquor and the like. It is moreover possible to add suitable precursors to the culture medium. The exact composition of the media compounds heavily depends on the particular experiment and is decided upon individually for each specific case. Information on the optimization of media can be found in the textbook "Applied Microbiol. Physiology, A Practical Approach" (Editors P.M. Rhodes, P.F. Stanbury, IRL Press (1997) pp. 53-73, ISBN 0 19 963577 3). Growth media can also be obtained from commercial suppliers, for example Standard 1 (Merck) or BHI (brain heart infusion, DIFCO) and the like.

All media components are sterilized, either by heat (20 min at 1.5 bar and 121°C) or by filter sterilization. The components may be sterilized either together or, if required, separately. All media components may be present at the start of the cultivation or added continuously or batchwise, as desired.

The culture temperature is normally between 15°C and 45°C, preferably at from 25°C to 40°C, and may be kept constant or may be altered during the experiment. The pH of the medium should be in the range from 5 to 8.5, preferably around 7.0. The pH for cultivation can be controlled during cultivation by adding basic compounds such as sodium hydroxide, potassium hydroxide, ammonia and aqueous ammonia or acidic compounds such as phosphoric acid or sulfuric acid. Foaming can be controlled by employing antifoams such as, for example, fatty acid polyglycol esters. To maintain the stability of plasmids it is possible to add to the medium suitable substances having a selective effect, for example antibiotics. Aerobic conditions are maintained by introducing oxygen or oxygen-containing gas mixtures such as, for example, ambient air into the culture. The temperature of the culture is normally 20°C to 45°C and preferably 25°C to 40°C. The culture is continued until formation of the desired product is at a maximum. This aim is normally achieved within 10 to 160 hours.

The fermentation broth can then be processed further. The biomass may, according to requirement, be removed completely or partially from the fermentation broth by separation methods such as, for example, centrifugation, filtration, decanting or a combination of these methods or be left completely in said broth. It is advantageous to process the biomass after its separation.

However, the fermentation broth can also be thickened or concentrated without separating the cells, using known methods such as, for example, with the aid of a rotary evaporator, thin-film evaporator, falling-film evaporator, by reverse osmosis or by nanofiltration. Finally, this concentrated fermentation broth can be processed to obtain the morphine present therein.

Not covered by the claims there is provided a plant cell transformed or transfected with a vector comprising a nucleic acid molecule according to the invention.

Preferably said vector is adapted for plant expression.

Preferably the nucleic acid molecule in the vector is under the control of, and operably linked to, an appropriate promoter or other regulatory elements for transcription in a host cell such as a microbial, (e.g. bacterial, yeast), or plant cell. The vector may be a bi-functional expression vector which functions in multiple hosts. In the case of genomic DNA this may contain its own promoter or other regulatory elements and in the case of cDNA this may be under the control of an appropriate promoter or other regulatory elements for expression in the host cell.

By "promoter" is meant a nucleotide sequence upstream from the transcriptional initiation site and which contains all the regulatory regions required for transcription. Suitable promoters include constitutive, tissue-specific, inducible, developmental or other promoters for expression in plant cells comprised in plants depending on design. Such promoters include viral, fungal, bacterial, animal and plant-derived promoters capable of functioning in plant cells. Constitutive promoters include, for example CaMV 35S promoter (Odell et al. (1985) Nature 313, 9810-812); rice actin (McElroy et al. (1990) Plant Cell 2: 163-171); ubiquitin (Christian et al. (1989) Plant Mol. Biol. 18 (675-689); pEMU (Last et al. (1991) Theor Appl. Genet. 81: 581-588); MAS (Velten et al. (1984) EMBO J. 3. 2723-2730); ALS promoter (U.S. Application Serial No. 08/409,297), and the like. Other constitutive promoters include those in U.S. Patent Nos. 5,608,149; 5,608,144; 5,604,121; 5,569,597; 5,466,785; 5,399,680, 5,268,463; and 5,608,142.

Chemical-regulated plant or microbial promoters can be used to modulate the expression of a gene in a plant or microbial cell through the application of an exogenous chemical regulator. Depending upon the objective, the promoter may be a chemical-inducible promoter, where application of the chemical induced gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88: 10421-10425 and McNellis et al. (1998) Plant J. 14(2): 247-257) and tetracycline-inducible and tetracycline-repressible promoters (see, for example, Gatz et al. (1991) Mol. Gen. Genet. 227: 229-237, and US Patent Nos. 5,814,618 and 5,789,156.

Where enhanced expression in particular tissues is desired, tissue-specific promoters can be utilised. Tissue-specific promoters include those described by Yamamoto et al. (1997) Plant J. 12(2): 255-265; Kawamata et al. (1997) Plant Cell Physiol. 38(7): 792-803; Hansen et al. (1997) Mol. Gen. Genet. 254(3): 337-343; Russell et al. (1997) Transgenic Res. 6(2): 157-168; Rinehart et al. (1996) Plant Physiol. 112(3): 1331-1341; Van Camp et al. (1996) Plant Physiol. 112(2): 525-535; Canevascni et al. (1996) Plant Physiol. 112(2): 513-524; Yamamoto et al. (1994) Plant Cell Physiol. 35(5): 773-778; Lam (1994) Results Probl. Cell Differ. 20: 181-196; Orozco et al. (1993) Plant Mol. Biol. 23(6): 1129-1138; Mutsuoka et al. (1993) Proc. Natl. Acad. Sci. USA 90 (20): 9586-9590; and Guevara-Garcia et al (1993) Plant J. 4(3): 495-50.

Particular of interest in the present context are nucleic acid constructs which operate as plant vectors. Specific procedures and vectors previously used with wide success in plants are described by Guerineau and Mullineaux (1993) (Plant transformation and expression vectors. In: Plant Molecular Biology Labfax (Croy RRD ed) Oxford, BIOS Scientific Publishers, pp 121-148. Suitable vectors may include plant viral-derived vectors (see e.g. EP194809). If desired, selectable genetic markers may be included in the construct, such as those that confer selectable phenotypes such as resistance to herbicides (e.g. kanamycin, hygromycin, phosphinotricin, chlorsulfuron, methotrexate, gentamycin, spectinomycin, imidazolinones and glyphosate).

Preferably said plant cell is from the family *Papaver.*

Preferably said plant cell is a *Papaver somniferum* cell.

According to an aspect of the invention there is provided a process for the extraction of codeine and/or related codeine alkaloids from a *Papaver* plant comprising the steps:
i) harvesting a plant or plant material prepared from a plant according to the invention;
ii) forming a reaction mixture of particulate plant material;
iii) solvent extraction of the reaction mixture to provide an alkaloid enriched fraction; and
iv) concentrating said alkaloid enriched fraction to provide an codeine enriched fraction.

In a preferred method of the invention said plant material comprises poppy straw and/or poppy latex.

Not covered by the claims is a method to produce a *Papaver* plant with elevated codeine content comprising the steps of:
i) mutagenesis of wild-type seed from a *Papaver* plant;
ii) cultivation of the seed in i) to produce first and subsequent generations of plants;
iii) obtaining seed from the first generation of plants and subsequent generations of plants;
iv) determining if the seed and /or plant material obtained through the germination of the seed from said first and subsequent generations of plants has altered nucleotide sequence and/or altered expression of said polypeptide comprising the steps of:
   a. obtaining a sample and analysing the nucleic acid sequence of a nucleic acid molecule selected from the group consisting of:
      1. a nucleic acid molecule comprising a nucleotide sequence as represented in SEQ ID NO: 1, 2, 3, 4, 5, 6, 7, 8, 9, 10, 11, 12, 13 or 14;
      2. a nucleic acid molecule that hybridises to a complementary strand of said nucleic acid molecule in a) under stringent hybridisation conditions and that encodes a polypeptide with codeine 3-O-demethylase activity; and optionally
   b. comparing the nucleotide sequence of the nucleic acid molecule in said sample to a nucleotide sequence of a nucleic acid molecule of the original wild-type plant.

Preferably said nucleic acid molecule is analysed by a method comprising the steps of:
i) extracting nucleic acid from said mutated plants;
ii) amplification of a part of said nucleic acid molecule by a polymerase chain reaction;
iii) forming a preparation comprising the amplified nucleic acid and nucleic acid extracted from wild-type seed to form heteroduplex nucleic acid;
iv) incubating said preparation with a single stranded nuclease that cuts at a region of heteroduplex nucleic acid to identify the mismatch in said heteroduplex; and
v) determining the site of the mismatch in said nucleic acid heteroduplex.

Preferably said nucleic acid molecule comprises the nucleotide sequence as set forth in SEQ ID NO: 44.

Methods to analyse expression of genes are known in the art. For example molecular techniques such as Polymerase Chain Reaction [PCR] i.e. that allow quantitative and qualitative measurement of mRNA expression and including nuclease protection methods, northern blot, quantitative primer extension, quantitative microarrays such as Serial Analysis of Gene Expression [SAGE], ELISA for measurement of protein levels. These are examples of methods available to the skilled person to analyse gene expression.
not covered by the claims is a plant obtained by the method according to the invention.

Not covered by the claim is the use of a nucleic acid molecule comprising or consisting of the nucleotide sequence set forth in SEQ ID NO 1 and/or SEQ ID NO: 2 and/or SEQ ID NO: 3 and/or SEQ ID NO: 4 and/or SEQ ID NO: 5 and/or SEQ ID NO: 6 and/or SEQ ID NO: 7, or a nucleic acid molecule which is to 99% identical to the nucleotide sequence set forth in SEQ ID NO: 1, SEQ ID NO: 2, and/or SEQ ID NO: 3 and/or, SEQ ID NO 4 and/or, SEQ ID NO 5, and/or SEQ ID NO 6 and/or SEQ ID NO 7 and encodes a polypeptide with codeine 3-O-demethylase activity as a means to identify the presence or absence of a gene that encodes said codeine 3-O-demethylase in a *Papaver* plant.

Preferably said nucleic acid molecule comprises or consists of all or part of the nucleotide sequence sequence set forth in SEQ ID NO: 44.

Not covered by the claims is a method to determine the presence or absence of a nucleic acid molecule encoding a polypeptide with codeine 3-O demethylase activity in a *Papaver* plant comprising:
i) obtaining a sample from a *Papaver* plant;
ii) extracting genomic DNA from the plant; and
iii) analyzing the genomic DNA for the presence of a nucleic acid molecule comprising or consisting of a nucleotide sequence set forth in SEQ ID NO 1 and/or SEQ ID NO: 2 and/or SEQ ID NO: 3. and/or SEQ ID NO: 4 and/or SEQ ID NO: 5 and/or SEQ ID NO: 6 and/or SEQ ID NO: 7.

Preferably said genomic DNA comprises or consists of a nucleotide sequence as set forth in SEQ ID NO: 44.

Not covered by the claims is a method of obtaining a *Papaver* plant with increased codeine content comprising:
i) crossing a *Papaver* plant according to the invention with a *Papaver* plant of different genotype to obtain seeds;
ii) germinate the obtained seeds and allow the germinated plant[s] to self-pollinate;
iii) germinate the seeds obtained in ii) and screen the germinated plants for increased codeine content and/or the presence and/or expression of one or more codeine 3-O-demethylase genes comprising a nucleotide sequence set forth in SEQ ID: NO: 1, SEQ ID NO: 2, SEQ ID NO: 3, SEQ ID NO: 4, SEQ ID NO: 5, SEQ ID NO: 6 or SEQ ID NO: 7 in the progeny of germinated plants;
iv) cross the plants identified in iii) above with said *Papaver* plant of different genotype in step i) to obtain seeds;
v) repeat step ii)-iv) at least once to provide a population of *Papaver* plants with increased codeine content in said plant of different genotype.

Preferably step iii) comprises plants comprising SEQ ID NO: 44.

Not covered by the claims is a *Papaver* plant obtained by the method according to the invention.

Throughout the description and claims of this specification, the words "comprise" and "contain" and variations of the words, for example "comprising" and "comprises", means "including but not limited to", and is not intended to (and does not) exclude other moieties, additives, components, integers or steps. "Consisting essentially" means having the essential integers but including integers which do not materially affect the function of the essential integers.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

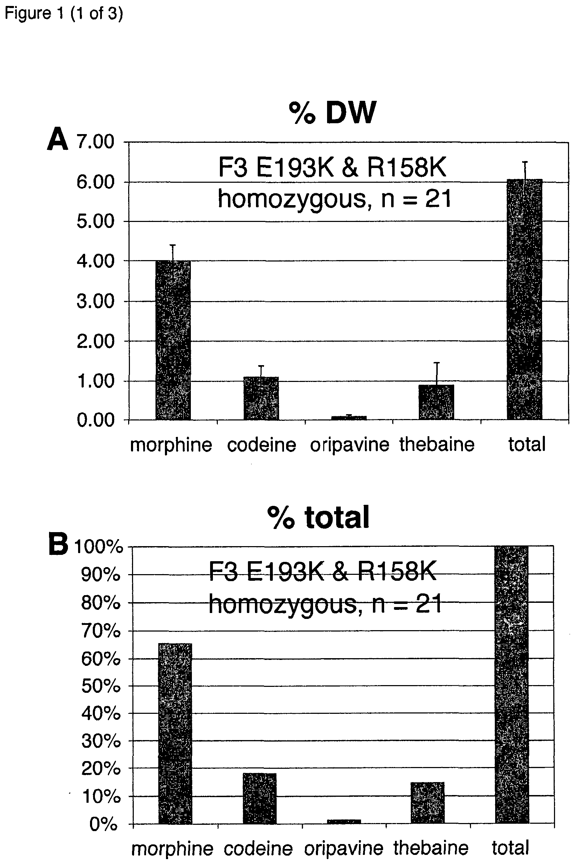

An embodiment of the invention will now be described by example only and with reference to the following figures and tables: Figure 1 A-F: Absolute and relative alkaloid content in F3 capsule material derived from crosses to morphine cultivar HM6. Figure A, Figure B, F3 capsules homozygous for R158K and E193K mutations/polymorphisms in their respective CODM copies; Figure C, Figure D, F3 capsules homozygous for CODM Q254*; Figure E, Figure F, control capsules from cultivar HM6. DW, dry weight; % total, relative amounts of the respective alkaloids of total main alkaloids (morphine, codeine, oripavine and thebaine);

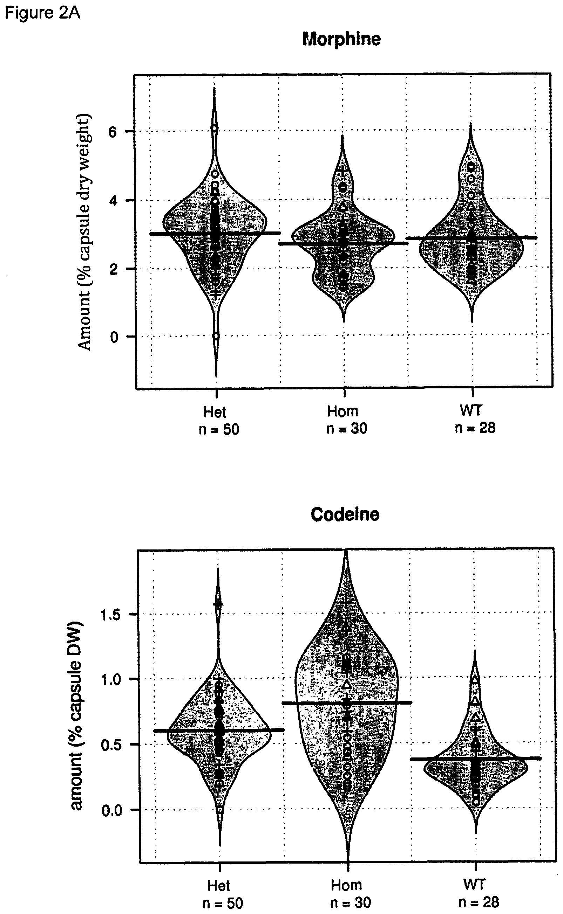

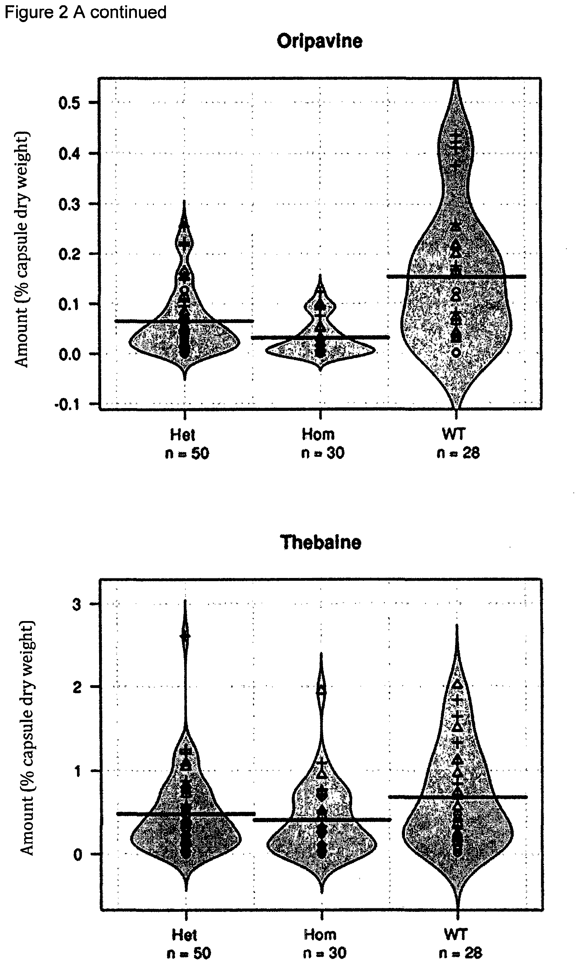

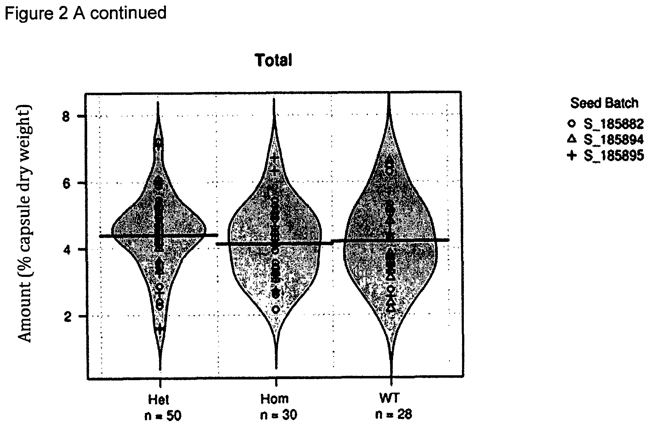

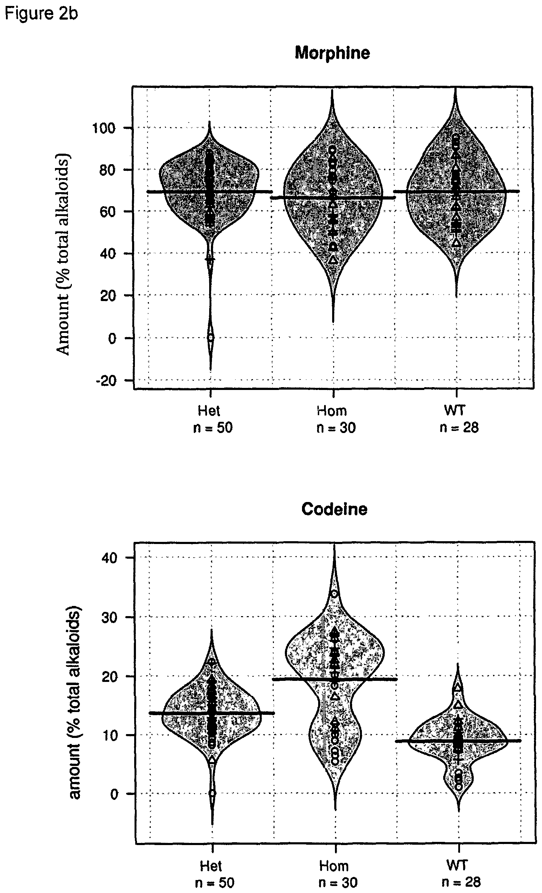

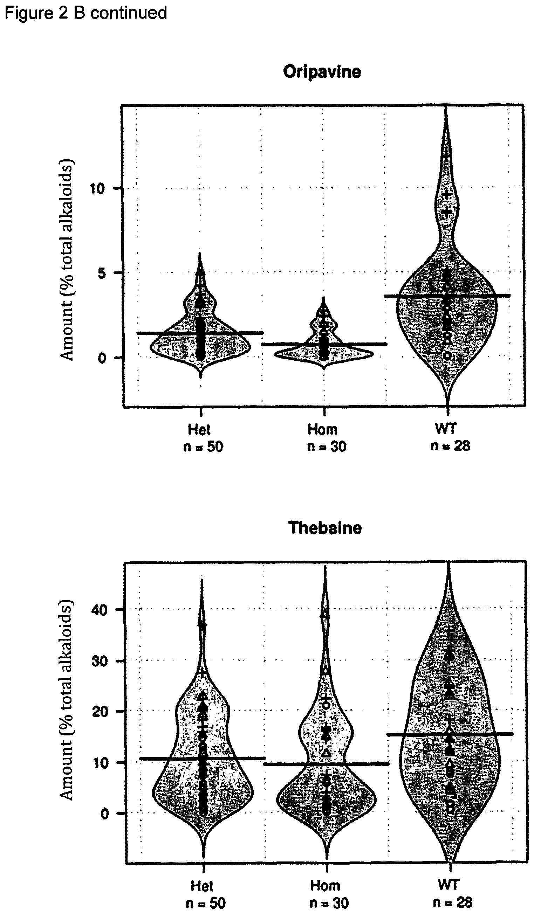

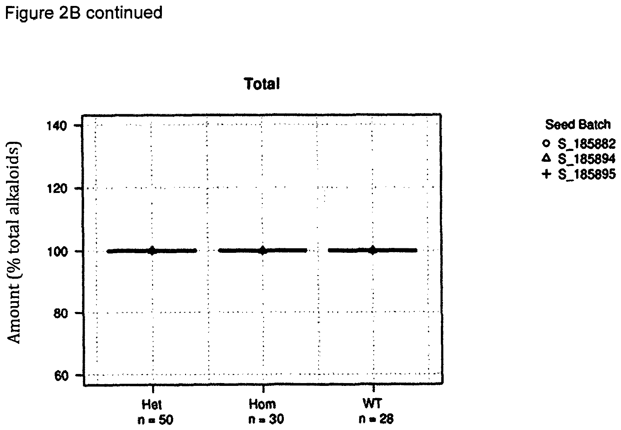

Figure 2: Absolute (A) and relative (B) alkaloid content in capsules of a BC₂F₂ population segregating the linked R158K and E193K mutations/polymorphism in their respective CODM copies. To set up the BC₂F₂ population the M7 generation of a high codeine individual harbouring the mutations/polymorphism was crossed three times to Sun Pharmaceutical Industries (Australia) cultivar HM6 as recurrent parent followed by a round of self-pollination. The BC₂F₂ was grown under glass for genotyping and metabolite profiling of capsules. Assignment to the genotypic classes homozygous (Hom), heterozygous (Het) and wild type (WT) was carried out as described in methods; seed batches o S_185882, Δ S_185894, + S_185895;

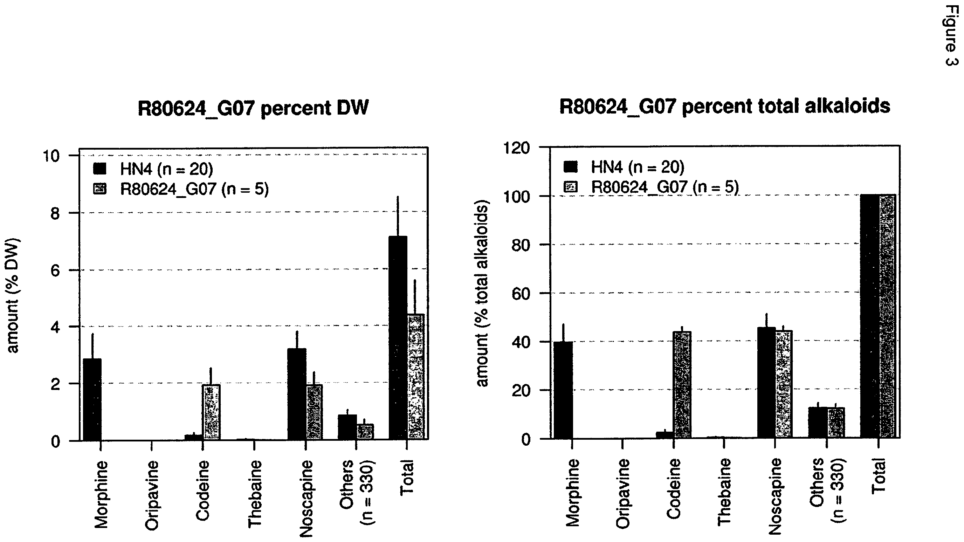

Figure 3: Absolute and relative alkaloid content in M3 capsules of the fast neutron mutagenised high codeine mutant R80624_G07 compared to control plants of the non-mutagenised wild type cultivar HN4. DW, dry weight;

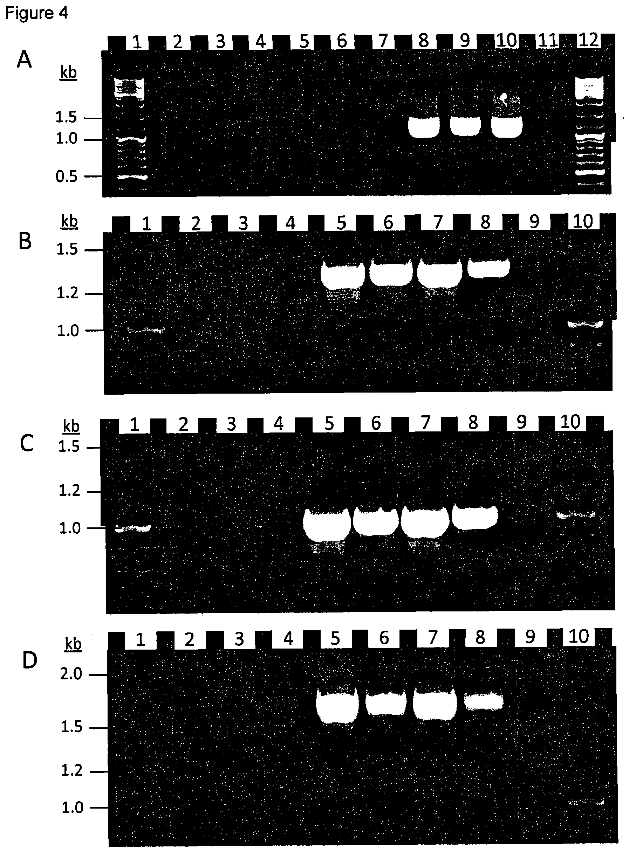

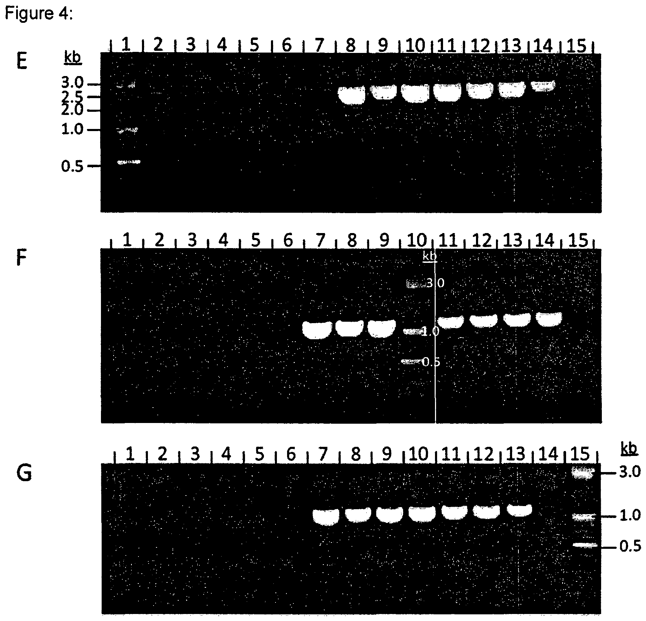

Figure 4: Amplification of CODM fragments from M4 indivduals of the fast neutron mutagenised codeine/noscapine mutant line and from non-fast neutron mutagenised control plants; A, primer pair 178/179, expected fragment size: 1296 bp; lane 1 and 12, size marker (GeneRuler DNA Ladder Mix, Thermo Fisher Scientific); lanes 2-7, M4 mutant plants Sd-701292, Sd-701293, Sd-701294, Sd-701302, Sd-701303, Sd-701304; lanes 8-10, control plants Sd-763815, Sd-763818, Sd-863819; lane 11, no template control. B, pimer pair 178/181, expected fragment size: 1347 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. C, pimer pair 180/179, expected fragment size: 1016 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. D, primer pair 180/168, expected fragment size: 1659 bp; lane 1 and 10, size marker (as above); lanes 2-4, M4 mutant plants Sd-701302, Sd-701303, Sd-701304; lanes 5-8, control plants Sd-763815, Sd-763818, Sd-863819, Sd-106305; lane 9, no template control. E, primer pair 167/168, expected fragment size: 2330 bp; lane 1, size marker (as above); lanes 2-8, loss of M4 mutant plants Sd-701292, Sd-701293, Sd-701294, Sd-701302, Sd-701303, Sd-701304; lanes 8-14, control plants Sd-763815, Sd-763818, Sd-863819, Sd-764849, Sd-764851, Sd-764857, Sd-106305; lane 15, no template control. F, primer pair 175/176, expected fragment size: 1122 bp; lane 10, size marker (as above); lanes 1-6, M4 mutant plants Sd-701292, Sd-701293, Sd-701294, Sd-701302, Sd-701303, Sd-701304; lanes 7- 9, control plants Sd-763815, Sd-763818, Sd-863819; lanes 11- 14 control plants Sd-764849, Sd-764851, Sd-764857, Sd-106305; lane 15, no template control. G, primer pair 175/177, expected fragment size: 1122 bp; lane 15, size marker (as above); lanes 1-7, M4 mutant plants Sd-701292, Sd-701293, Sd-701294, Sd-701302, Sd-701303, Sd-701304; lanes 7-13, control plants Sd-763815, Sd-763818, Sd-863819, Sd-764849, Sd-764851, Sd-764857, Sd-106305; lane 14, no template control.

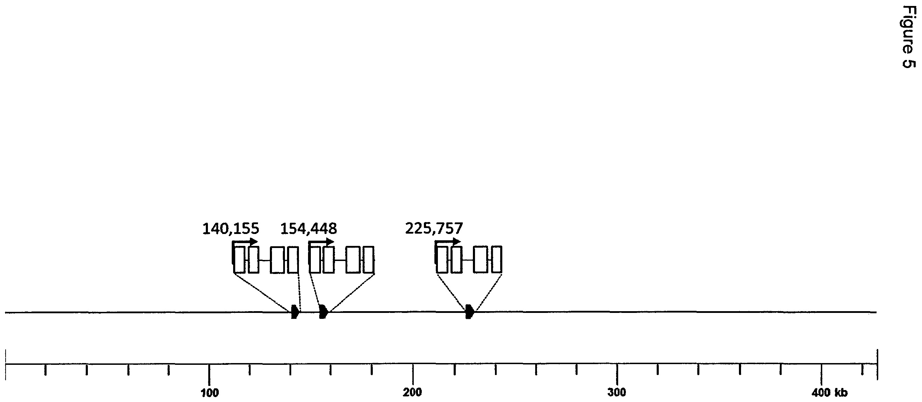

Figure 5: Illustrates the genomic arrangement of CODM genes in opium poppy. The structure and position of the three CODM genes are shown above the central black line, which represents 426.6 kb of genomic sequence. Exons are represented by solid grey boxes and introns by fine black lines. Arrows indicate the 5' to 3' orientation of each gene. The number above the gene structure indicates the start position of each gene (first base of transcriptional start codon) within the genomic sequence comprising the CODM genes (SEQ ID NO44).

Sequence identifiers of sequences supplied (an overview is shown in Table 1):
SEQ ID NO 1: genomic sequence of CODM gene variant
SEQ ID NO 2: genomic sequence of CODM gene variant
SEQ ID NO 3: genomic sequence of CODM gene variant
SEQ ID NO 4: genomic sequence of CODM gene variant
SEQ ID NO 5: genomic sequence of CODM gene variant
SEQ ID NO 6: genomic sequence of CODM gene variant
SEQ ID NO 7: genomic sequence of CODM gene variant
SEQ ID NO 8 deduced coding sequence of CODM gene variant SEQ ID NO 1
SEQ ID NO 9 deduced coding sequence of CODM gene variant SEQ ID NO 2
SEQ ID NO 10 deduced coding sequence of CODM gene variant SEQ ID NO 3
SEQ ID NO 11 deduced coding sequence of CODM gene variant SEQ ID NO 4
SEQ ID NO 12 deduced coding sequence of CODM gene variant SEQ ID NO 5
SEQ ID NO 13 deduced coding sequence of CODM gene variant SEQ ID NO 6
SEQ ID NO 14 deduced coding sequence of CODM gene variant SEQ ID NO 7
SEQ ID NO 15 deduced amino acid sequence of CODM gene variant SEQ ID NO 8
SEQ ID NO 16 deduced amino acid sequence of CODM gene variant SEQ ID NO 9
SEQ ID NO 17 deduced amino acid sequence of CODM gene variant SEQ ID NO 10 and 13
SEQ ID NO 18 deduced amino acid sequence of CODM gene variant SEQ ID NO 11, 12 and 14.
SEQ ID NO: 44 genomic cluster comprising CODM genes

**Table 1: Overview of CODM copies/alleles amplified from BC₂F₂ homozygous for mutations/polymorphisms R158K and E193K. *The positions of the nucleotide variations observed refer to the position within the respective genomic sequences (SEQ ID NO 1-7)**

| | **Position* and type of variation** | | | | | | | | | | | |
|---|---|---|---|---|---|---|---|---|---|---|---|---|
| **genomic CODM SEQ ID NO** | **177 [A->G] N11D** | **701 [C->A] R158K** | **1106 [G->A] E193K** | **798 [T indel] intronic** | **116 [G->A] synon.** | **1186 [T->C] synon.** | **1601 [G->T] synon.** | **1737 [T->C] 3'UTR** | **1838 [G->A] 3'UTR** | **1982-3 [TT indel] 3' UTR** | **SEQ ID NO of deduced coding sequence** | **SEQ ID NO of deduced amino acid sequence** |
| SEQ ID NO 1 | √ | | | | | | √ | | | TT insert. | 8 | 15 |
| SEQ ID NO 2 | | | √ | | | √ | √ | | | | 9 | 16 |
| SEQ ID NO 3 | | √ | | | | | √ | | | TT insert. | 10 | 17 |
| SEQ ID NO 4 | | | | √ (T del) | √ | | | | | | 11 | 18 |
| SEQ ID NO 5 | | | | | | | √ | | √ | TT insert. | 12 | 18 |
| SEQ ID NO 6 | | √ | | | | | | √ | | | 13 | 17 |
| SEQ ID NO 7 | | | | | | | | √ | | TT insert. | 14 | 18 |
| synon.= synonymous; insert. = insertion | | | | | | | | | | | | |

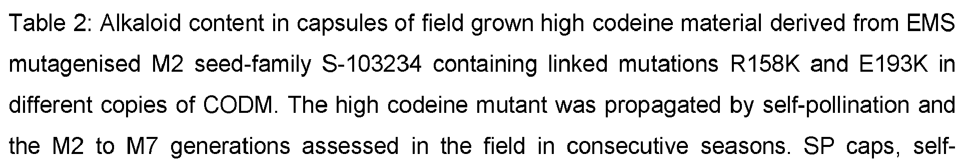

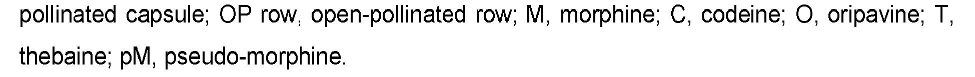

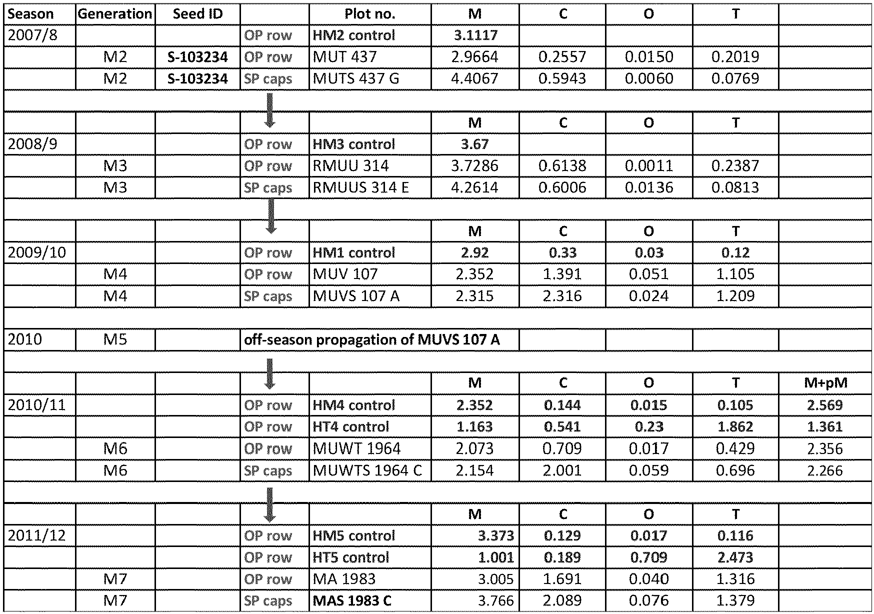

### Materials and Methods

### EMS mutagenesis for forward and reverse genetic screening

Prior to mutagenesis, M0 seeds from *P. somniferum* Sun Pharmaceutical Industries (Australia) cultivar High Morphine 2 (HM2) were soaked in 0.1% (w/v) potassium chloride solution for approximately 24 hours. The seed was then imbibed for three (treatment B) or five hours (treatment C) with 200 mM ethyl methanesulfonate (EMS) (in 100 mM sodium hydrogen phosphate buffer, pH 5.0, supplemented with 700 mM dimethyl sulfoxide). The treated M1 seed was washed twice for 15 minutes each with 100 mM sodium thiosulphate solution and then twice for 15 minutes each with distilled water. A volume of 10 mL of the respective solutions was used per 1000 seeds and all steps were carried out on a rocking platform. The washed M1 seed was dried overnight on Whatman 3MM Blotting paper (Whatman/GE Healthcare Life Sciences, Little Chalfont, UK) and sown the following morning on top of a mixture of John Innes no 2 compost, vermiculite and perlite (4:2:1), then covered with a thin layer of compost. Seedlings were transplanted three to four weeks after germination into larger pots and grown in the glasshouse until maturity. Dried capsules were harvested by hand from the M1 population once capsules had dried to approximately 10% moisture on the plant. M2 seed was manually separated from the capsule, providing the M2 seed families that were used in the field-based forward screening and reverse genetic screening described below.

### Field-based forward screening of EMS-mutagenized lines

M2 seed was sown in in the field in Tasmania in the 2007/2008 growing season. M2 seed lines from the EMS mutagenized population were hand sown in 3m long rows. After germination, plants were thinned to 75 mm apart. Typically, 4 plants (designated A, B, C, D) per M2 line were self-pollinated (two immature buds per plant selected) by securing a linen bag over the bud. Open-pollinated rows were harvested at ~ 11% moisture, and assayed (as described below) for morphine (M), codeine (C), oripavine (O) and thebaine (T) content. Analysis of selected self-pollinated capsules from an M2 plants indicated higher assays of codeine. M3 to M7 seeds derived from a selected self-pollinated M2 capsule were sown in the following growing seasons in Tasmania to confirm this phenotype in both open-pollinated and self-pollinated capsules. Typically 4 plants (designated A, B, C, D) were self-pollinated per generation as described above. Sun Pharmaceutical Industries (Australia) commercial cultivars were grown alongside the M2 to M7 lines as a comparison. Depending on the season, they included Sun Pharmaceutical Industries (Australia) HIGH MORPHINE CULTIVARs HM1, HM2, HM3, HM4 and HM5 and Sun Pharmaceutical Industries (Australia) HIGH THEBAINE CULTIVARs HT4 and HT5. These cultivars do produce the major morphinans morphine, codeine, oripavine and thebaine but lack the biosynthetic capacity to produce noscapine.

### Poppy straw analysis of field-based forward screening

Poppy capsules were harvested from the respective mutant lines by hand once capsules had dried to approximately 10% moisture on the plant. The seed was manually separated from the capsule, and capsule straw material (poppy straw) was then shipped to the Sun Pharma's extraction facility in Port Fairy, Australia.

The poppy straw samples were ground in a Retsch Model MM400 ball mill into a fine powder. Two gram samples of ground poppy straw (2 ± 0.003 g) were extracted in 50 mL of a 10% acetic acid solution. The extraction suspension was shaken on an orbital shaker at 200rpm for a minimum of 10 minutes then filtered to provide a clear filtrate. The final filtrate was passed through a 0.22 µm filter prior to analysis.

The solutions were analysed using a Waters Acquity UPLC system (Waters Corporation, Milford, MA, USA) fitted with a Waters Acquity BEH C18 column, 2.1mm × 100mm with 1.7 micron packing. The mobile phase used a gradient profile with eluent A consisting of 0.1% Trifluoroacetic acid in deionised water and eluent B consisting of 100% Acetonitrile. The mobile phase gradient conditions used are as listed in Table 3, the gradient curve number as determined using a Waters Empower chromatography software package. The flow rate was 0.6 mL per minute and the column maintained at 45 degrees centigrade. The injection volume was 1 µL injection volume and the alkaloids were detected using a UV detector at 285 nm.

The loss on drying (LOD) of the straw was determined by drying in an oven at 105 degrees centigrade for 16-20 hours. Alkaloid concentrations for morphine, pseudomorphine, codeine, thebaine, oripavine and noscapine were quantified using external standard calibration and the results calculated on a dry weight basis. Reticuline was quantified using the thebaine calibration (assuming the thebaine response) and results calculated on a dry weight basis (% weight relative to thebaine; %WRT).

**Table 3: Gradient Flow Program**

| **TIME (minutes)** | **% Eluent A** | **% Eluent B** | **Flow (mL/min)** | **Curve No** |
|---|---|---|---|---|
| 0.00 | 95.0 | 5.0 | 0.60 | INITIAL |
| 0.80 | 90.0 | 10.0 | 0.60 | 6 |
| 3.40 | 75.0 | 25.0 | 0.60 | 3 |
| 3.60 | 95.0 | 5.0 | 0.60 | 6 |
| 4.00 | 95.0 | 5.0 | 0.60 | 11 |

Typical retention times are as follows:

| **Compound** | **Retention Time (minutes)** |
|---|---|
| Morphine | 1.14 |
| Pseudomorphine | 1.26 |
| Codeine | 1.69 |
| Oripavine | 1.80 |
| Reticuline | 2.05 |
| 10-Hydroxythebaine | 2.32 |
| Thebaine | 2.53 |
| Noscapine | 3.16 |

### Establishment of a TILLING population for reverse genetic screening

A subset of the EMS Band C populations were grown in the glasshouse to establish a TILLING population. One M2 sibling per M2 seed family was grown for DNA extraction. DNA was extracted from four week old seedlings as described below. In total DNA was obtained from 4,146 M2 plants (Table 4). The M2 seedlings were sacrificed after DNA sampling. Any mutations identified in an M2 plant were retrieved by re-sowing and re-screening the respective M2 siblings.

DNA for the TILLING population was quantified using Hoescht dye 33258 and normalised to 10 ng/µL in TE using the TECAN Freedom Evo^{®} 100 platform (TECAN, Switzerland). DNA samples were pooled four-fold prior to screening.

**Table 4: TILLING populations**

| **EMS treatment** | **No. of M2 seed families** | **M2 seed families identifiers** | **No of germinating M2 seed lines (% germination)** |
|---|---|---|---|
| B | 4779 | S-100001 to S-102800, S-102831to S-103227, S-103647 to S-105228 | 3807 (79%) |
| C | 419 | S-103228 to S-103646 | 339 (56%) |

### Preparation of genomic DNA

For genomic DNA extraction 30-50 mgs of leaf material was collected from 4-6 week old glasshouse-grown seedlings. Genomic DNA was extracted using the BioSprint 96 Plant kit on the BioSprint 96 Workstation (Qiagen, Crawley, UK) according to the manufacturer's protocol. Extracted DNA was spectrometrically quantified on the NanoDrop^{®} (Thermo Scientific, UK) and normalized to 5 or 10 ng/ul.

### Cloning of the CODM genes from genomic DNA

Full-length CODM gene sequences were amplified from MAS1983 C mutant homozygous line using a high fidelity PCR enzyme. PCR reactions containing 1x Phusion HF buffer, 0.2mM dNTPs, 0.5µM) forward (primer id 167) and reverse (primer id 168) primer (Table 5) and 0.4U Phusion DNA polymerase (ThermoFisher) in a total volume of 20µl were run on a Tetrad thermocycler (Bio-Rad) under the following conditions: 98°C for 30 seconds, followed by 10 cycles of 98°C for 10 seconds, 65°C decreasing to 60°C by 0.5°C per cycle for 30 seconds, 72°C for 1 minute, followed by 28 cycles of 98°C for 10 seconds, 60°C for 30 seconds, 72°C for 1 minute, followed by 72°C for 10 minutes, then a hold at 7°C. PCR products were checked on 1% agarose gel, extracted by Wizard SV PCR extraction kit (Promega) according to the manufacturer's instructions, and the concentration determined spectrometrically by on the NanoDrop^{®} (Thermo Scientific).

Purified CODM PCR product was cloned using the CloneJET PCR Cloning Kit (Thermo Scientific) according to the manufacturer's protocol, and transformed into SoloPack Competent Cells (StrataClone) according to the manufacturer's protocol.

Ten single colonies positive for the CODM insert were selected by colony PCR. Plasmids were extracted using the QlAprep Spin Miniprep kit (Qiagen) and used for Sanger-sequencing. Primers for amplification and sequencing are shown in Table 5. Among the ten clones sequenced, seven different CODM gene sequences were identified (SEQ IDs 1-7, Table 5). The transcribed and coding sequences were deduced by comparison with the published CODM cDNA sequence (GenBank: GQ500141) resulting in SEQ IDs 8-14 (coding sequences) and SEQ IDs 15-18 (amino acid sequences).

### Amplification of CODM fragments from genomic DNA

CODM fragment PCR amplifications were carried out on genomic DNA from M4 individuals derived from the high codeine/noscapine fast neutron mutagenised mutant material R80624_G07 (M4 mutant siblings: Sd-701292, Sd-701293, Sd-701294, Sd-701302, Sd-701303, Sd-701304; control plants Sd-763815, Sd-763818, Sd-863819, Sd-764849, Sd-764851, Sd-764857, Sd-106305) as well as on non-fast neutron mutagenised material derived from Sun Pharmaceutical Industries (Australia) morphine cultivar HM2.

PCR reactions containing 5x GoTaq Flexi buffer, 0.2mM dNTPs, 1.0 µM forward and reverse primer (Tables 5 and 6), 1U GoTaq Flexi DNA polymerase (Promega, M8305), 1.5 mM MgCl₂ and 10 ng of template DNA in a total volume of 10µl were run on a Tetrad thermocycler (Bio-Rad) under the following conditions: 94°C for 2 minutes, followed by 10 cycles of 94°C for 15 seconds, 65°C decreasing to 60°C by 0.5°C per cycle for 30 seconds, 72°C for 1-1.5 minute (depending on fragment length), followed by 25 cycles of 94°C for 15 seconds, 60°C for 30 seconds, 72°C for 1-1.5 minute (depending on fragment length), followed by 72°C for 10 minutes, then a hold at 7°C. PCR products were analysed on 1-1.5% agarose gels. Primers and primer combinations used for amplification are shown in Tables 5 and 6.

### Reverse genetic screening of Codeine 3-O-demethylase (CODM)

CODM we amplified from pool plates using CODM F9/R5 (Table 5). This primer pair would amplify all three known CODM copies simultaneously. 10µl PCR reactions were set up with 0.1µl GoTaq Flexi DNA polymerase (Promega, M8305), 0.6µl MgCl₂ (25mM), 2µl GoTaq Flexi colourless buffer, 0.2µl dNTPs (New England Biolabs, N0447), 0.5µl of each primer (10µM), 4.1µl HyClone molecular biology grade water (Thermo Scientific, SH3053801) and 2µl (20ng) of template DNA.

The PCR conditions were as follows: 95°C 2min, 10 cycles of (94°C for 20sec, annealing temperature of 67-62°C (-0.5°C per cycle) for 30 sec , 72°C for 75 sec) 37 cycles of (94°C for 20sec, 62°C for 30 sec , 72°C for 75 sec, final extension at 72°C for 5min.

**Table 5: Primers used for cloning, fragment amplification and sequencing**

| **Primer_id** | **Primer purpose** | **sequence (5'->3')** |
|---|---|---|
| 167 | CODM cloning from genomic DNA | GTGGTTGCTCAGACCCTTCGT |
| 168 | CODM cloning from genomic DNA | AGCACCACCACCAAGCCTTC |
| 169 | Sequencing | AGAAACCAACCAACGTACCCTAGC |
| 175 | Sequencing, CODM fragment amplification | AACCATGGAGTCGACGCTTT |
| 176 | CODM amplification | TCTCTGGTGTGACCAAGCTC |
| 177 | CODM amplification | TCTCTGGTGTGACCAAGCTA |
| 178 | Sequencing, CODM fragment amplification | CTCACGCTTGCAGAAATTCC |
| 179 | Sequencing, CODM fragment amplification | CTCGACGCTACGGTAAATCC |
| 180 | CODM fragment amplification | GTTAACCATGGAGTCGACGC |
| 181 | CODM fragment amplification | AAATGTCGCGATTGAGAGCC |
| CODMseqF1 | Sequencing | TCCGGGTTTGTGAATTGCAT |
| CODMseqR1 | Sequencing | ATTCCAGTGTCTCCCTGCAT |
| pJET1.2 Forw | Sequencing | CGACTCACTATAGGGAGAGCGGC |
| pJET1.2 Rev | Sequencing | AAGAACATCGATTTTCCATGGCAG |
| CODM_F9 | TILLING | CGACGCTTTACTGATGGACA |
| CODM_R5 | TILLING | CATCCACGAAATTTAAGCACAA |

**Table 6: Primer combination for amplification of genomic CODM fragments shown in Figure 4**

| Fragment | Forward primer ID | Reverse primer ID | Expected fragment size | Figure |
|---|---|---|---|---|
| 1 | 178 | 179 | 1296 | Figure 4A |
| 2 | 178 | 181 | 1347 | Figure 4B |
| 3 | 180 | 179 | 1016 | Figure 4C |
| 4 | 180 | 168 | 1659 | Figure 4D |
| 5 | 167 | 168 | 2330 | Figure 4E |
| 6 | 175 | 176 | 1122 | Figure 4F |
| 7 | 175 | 177 | 1122 | Figure 4G |

This was followed immediately by heteroduplex formation as follows: 99°C for 10min, 70°C for 20sec (-0.3°C per cycle) × 70, old at 4°C. After heteroduplex formation, the PCR products were diluted five-fold with sterile dH₂O. 2µl of this diluted PCR product was treated with 2µl of working concentration dsDNA Cleavage Enzyme (Advanced Analytical Technologies Inc. (AATI), FS-CLKENZ) in the heteroduplex digestion reaction. Mutations were detected using the Mutation Discovery Kit (AATI, DNF-920-K0500T) on the Fragment Analyzer^{™} machine (AATI) according to the manufacturer's instructions for the Mutation Detection Kit. The machine was fitted with a 96-Capillary Array Cartridge (Fluorescence), 55cm effective/80cm total length, 50µm ID (AATI, A2300-9650-5580). Running conditions were as follows:

### Full condition:

Prerun: 12kV 30sec
Marker voltage injection (35bp and 5000bp): 7.5kV 15sec
Sample voltage injection: 7.5kV 45sec
Separation: 12kV 75min

A mutation in a pool resulted in heteroduplex formation following PCR and subsequent cleavage. Mutations were detected if two small fragments of DNA whose total sizes corresponded to the full length PCR product size were observed in a lane.

### Generation of a F₃ population for the CODM Q254* truncation mutation

A G:C to A:T transition mutation introducing a premature stop codon at amino acid residue Q254 (Q254*) was identified in a CODM copy in the EMS-mutagenised M2 seed family S-104530. The mutant line was crossed to Sun Pharmaceutical Industries (Australia) cultivar HM6 and the F, generation self-pollinated to generate segregating F₂ which was genotyped for Q254*. F₃ material derived from a homozygous F₂ mutant plant was field trialed in Tasmania, QC-genotyped and the alkaloid profile assessed.

### Generation of a BC₂F₂ population segregating CODM R158K and E193K mutations

High codeine mutants identified in the EMS forward screen found to contain CODM R158K and E193K mutations (SEQ ID NO 2, 3, 6, 9, 10, 13, 16, 17) were crossed three times to recurrent parent Sun Pharmaceutical Industries (Australia) cultivar HM6 followed by a round of self-pollination to generate a BC₂F₂ population segregating R158K and E193K in their respective CODM copies. The polymorphisms/mutations were tracked through the generations by genotyping. In addition to setting up BC₁ crosses, F₁ material was also self-pollinated to generate segregating F₂ populations. F₃ material derived from F₂ plants homozygous for R158K and E193K was assessed in the field in Tasmania. The segregating BC₂F₂ population was grown, genotyped and phenotyped in the glasshouse.

### Genotyping of CODMb Q254* mutation

The CODMb Q254* mutation was genotyped by means of allele-specific PCR. 10µl PCR reactions were set up in wells of a 96 well PCR plate (Starlabs, 11402-9800) with 0.5U GoTaq Flexi DNA polymerase (Promega, M8305), 1.5 mM MgCl₂, 1 × GoTaq Flexi colourless buffer, 200 µM dNTPs (New England Biolabs, N0447), 500 nM of each primer (Table 7) and 20ng of template DNA.

**Table 7: Primer sequences for allele-specific PCR assays to genotype mutation Q254***

| **Marker** | **Wild-type Primer_Allele (5'->3')** | **Mutant allele Primer_Allele (5'->3')** | **Primer_Common (5'->3')** |
|---|---|---|---|
| CODM_Q254* | GAAATCCACCTCTCTT CTTTTCTTATTCG | GATTGAAATCCACCTCT CTTCTTTTCTTATTAA | TGTCAGATGGAAAATA TACTGAAGTTGCA |

PCR conditions were as follows: 95°C for minutes, 35 cycles of 95°C for 30 seconds, 59°C for 30 seconds, 72°C for 30 seconds, followed by a final extension at 72°C for 5 minutes.

PCR products were visualised on 1.5% w/v agarose gel.

### Genotyping of mutations/polymorphisms R158K and E193K

KASP genotyping assays (LGC Genomics) were developed to track the mutations leading to the E193K and R158K amino acid substitutions in CODMa/c and CODMb, respectively. Allele-specific and common primers used in the assays are shown in Table 8.

PCR reactions containing 12.5 ng DNA, 3% DMSO , 1x Master Mix (KASP4.0 low ROX, LGC Genomics) and 1x Primer Mix (LGC Genomics) in a total volume of 5µl were run on a Hydrocycler (LGC) PCR machine under the following conditions: 94°C for 15 minutes, followed by 10 cycles of 94°C for 20 seconds and 65°C decreasing to 57°C for 1 minute, followed by 38 cycles of 94°C for 20 seconds and 57°C for 1 minute. Following completion of the KASP PCR, fluorescent reading of PCR products was performed on a ViiA7 (Applied Biosystems). Cluster analysis was performed with the Applied Biosystems ViiA(TM) 7 Software v.1.2.1. Despite interrogating the same mutation/polymorphism in two different CODM variants (Table 1) typically three distinct genotypic classes were observed for marker CODM_R158K in segregating populations. The classes were assigned to homozygous for the mutant allele (AA), homozygous for the wild type allele (GG) and heterozygous (AG) based on controls (Homozygous class: DNA from individuals homozygous for SEQ ID NO: 3 and 6, heterozygous class: DNA from individuals homozygous for SEQ ID NO: 3 and 6 mixed with equal amounts of HM6 DNA, wild type class: HM6 DNA.

**Table 8: Primer sequences of KASP genotyping assays for R158K and E193K mutations/polymorphisms**

| **KASP_I D** | **Wild-type Primer_AlleleFAM (5'->3')** | **Mutant allele Primer_AlleleHEX (5'->3')** | **Primer_Common (5'->3')** |
|---|---|---|---|
| CODM _ E193K | CAACGGTTGTCTTTG AGATGTTGG | ATCAACGGTTGTCTTT GAGATGTTGA | CCCATCTTCAAATAAGT CTGTCATACCTT |
| CODM _ R158K | AGTTCTGGAAACAAA TGAGGCTTCC | GAGTTCTGGAAACAA ATGAGGCTTCT | GTTTAGCATGTTAAGTC TTCCTCTCCATT |

### Fast Neutron Deletion Mutagenesis and glasshouse-based forward screening

Fast neutron mutagenesis (FNM) was carried out by the HAS Centre for Energy Research, Radiation Protection Department, H-1121 Budapest, Konkoly Thege út 29-33, X. epulet, Hungary. About 40 g of seed of the Sun Pharmaceutical Industries (Australia) cultivar High Noscapine 4 (HN4) were exposed to 20 Gy (beam time 50 min). The M1 generation was self-pollinated. One M2 plant per M2 seed family was grown. Latex was collected from 9 week old M2 seedlings and analysed. M2 plants displaying an altered alkaloid composition in the latex compared to HN4 controls were grown to maturity to confirm the phenotype in dry M2 capsule material. M3 seed was collected from M2 plants with confirmed phenotypes. Five M3 plants were then grown per M2 mutant plant to confirm the heritability of the phenotype in mature dry M3 capsule material. The M1 to M3 generations were all grown in the glasshouse.

### Leaf latex and dry capsule analysis of glasshouse grown material

Latex was collected from 9 week old plants from cut petioles, with a single drop dispersed into 500 µL of 10% acetic acid. This was diluted 10× in 1% acetic acid to give an alkaloid solution in 2% acetic acid for further analysis. Capsules were harvested from the same plants used for latex analysis and single capsules were ground to a fine powder in a ball mill (Model MM04, Retsch, Haan, Germany). Samples of ground poppy straw were then weighed accurately to 10 ±0.1 mg and extracted in 0.5 ml of a 10% acetic acid solution with gentle shaking for 1h at room temperature. Samples were then clarified by centrifugation and a 50 µl subsample diluted 10× in 1% acetic acid to give an alkaloid solution in 2% acetic acid for further analysis.

All solutions were analysed using a Waters Acquity UPLC system (Waters Ltd., Elstree, UK) fitted with a Waters Acquity BEH C18 column, 2.1 mm × 100 mm with 1.7 micron packing. Themobile phase used a gradient profile with eluent A consisting of 10 mM ammonium bicarbonate of pH 10.2 and eluent B methanol. The mobile phase gradient conditions used are as listed in the table below with a linear gradient. The flow rate was 0.5 ml per minute and the column maintained at 60°C. The injection volume was 2 µl and eluted peaks were ionised in positive APCI mode and detected within 5 ppm mass accuracy using a Thermo LTQ-Orbitrap. The runs were controlled by Thermo Xcalibur software (Thermo Fisher Scientific Inc., Hemel Hempstead,UK).

| **TIME (minutes)** | **% Eluent A** | **% Eluent B** | **Flow (mL/min)** |
|---|---|---|---|
| 0.0 | 98 | 2 | 0.50 |
| 0.2 | 98 | 2 | 0.50 |
| 0.5 | 60 | 40 | 0.50 |
| 4.0 | 20 | 80 | 0.50 |
| 4.5 | 20 | 80 | 0.50 |

All data analysis was carried out using the R programming language and custom scripts. Standards for morphine, oripavine, codeine, thebaine and noscapine were used to identify and quantify these alkaloids using exact mass, retention time and peak areas for the pseudomolecular ion. Other putative alkaloid peaks were quantified by their pseudomolecular ion areas relative to the thebaine standard. For putative alkaloids, the Bioconductor rcdk package (Guha, (2007) J.Stat. Software 6(18)) was used to generate pseudomolecular formulae from exact masses within elemental constraints C = 1 100, H = 1 200, O = 0 200, N = 0 3 and mass accuracy < 5 ppm. The hit with the lowest ppm error within these constraints was used to assign a putative formula.

### Bacterial Artificial Chromomsome (BAC) library construction and screening.

High Noscapine cultivar cvs 1 was used to generate a Bacterial Artificial Chromomsome (BAC) library as described in Winzer et al. ((2012), Science 336:1704-8).

A 703 bp fragment located in the CODM promoter region (Raymond M, "Isolation and characterization of latex-specific promoters from Papaver somniferum", Master thesis, Virginia Polytechnic Institute and State University, Blacksburg,VA) was amplified with primers AAAATCCGCCCTCCATGC (forward) and CCGACTTTGGCCCACTTGT (reverse) using a PCR digoxigenin (DIG) synthesis kit (Roche Applied Science, Indianapolis, IN) according to the manufacturer's instruction to obtain the DIG-labeled screening probe. Screening of the BAC library was performed as described Winzer et al. ((2012), Science 336:1704-8) and resulted in the identification of 10 positive BAC clones, namely BAC33_D07, BAC86_F04, BAC89_C05, BAC109_H06, BAC129_K11, BAC152_G13, BAC158_A11, BAC185_L02, BAC195_N12 and BAC230_D02.

### BAC Sequencing and Sequence Assembly.

Each BAC was sequenced on two sequencing platforms: Paired-end sequencing was performed on the Illumina HiSeq platform (Illumina,Inc, San Diego, CA).

In addition, each BAC clone was sequenced using a MinION sequencer (Oxford Nanopore Technologies Ltd, Oxford, UK). Purified BACs were minimally fragmented using 20 second treatments with NEBNext^{®} dsDNA Fragmentase^{®} (New England Biolabs Inc., Ipswich, MA), and sequencing libraries prepared using Oxford Nanopore Technologies sequencing kit SQK007 with native barcoding expansion pack EXP-NDB002. Briefly, single stranded nicks in DNA were repaired using NEBNext^{®} FFPE DNA repair mix prior to end repair and dA tailing using the NEBNext^{®} Ultra^{™} II End Repair/dA-Tailing Module. Unique barcode sequences were ligated onto fragments for each BAC, before pooling 3-4 BACs per library. Sequencing adapters (including a hairpin adapter to allow for 2D sequencing) were ligated onto the ends of fragments, along with an adapter-binding tether protein. Fragments where tether protein was bound were purified using MyOne^{™} C1 steptavidin beads (Invitrogen/Thermo Fisher Scientific, UK). Libraries were then run on MinION R9 flowcells using a 48 hour sequencing protocol, and base calling and demultiplexing was perfomed using Metrichor's EPI2ME platform. Reads that passed 2D quality checks were split according to barcode for further analysis.

### Sequence Assembly

Raw MiniON reads of each BAC clone were assembled with CANU v1.3 software (Koren et al. (2016) bioRxiv, doi.org/10.1101/071282) and insert boundaries were identified with the positions of vector sequences and cross-reference of overlapping BAC clones. These initial assemblies were further corrected with the NANOPOLISH software. The high quality Illumina paired-end short reads were then mapped with BWA software (Li and Durbin (2009) Bioinformatics, 25: 1754-60) to the resulting MinION assemblies, which were used as the scaffolding reference. Consensus reference were generated with the mapped alignment and indels were corrected according to the variation analyses of the alignment. The corrected consensus was then used as a new reference in a reiterative process until no further improvement could be achieved. This assembly method has shown to give a 99.2 % base identity to a previously assembled and published BAC sequence (BAC164_F07, gene bank accession: JQ659012). The overlapping BACs were then combined to give a continuous 426 KB CODM genome fragment.

### Example 1

### Multiple copies of CODM exist in the genome of opium poppy

Copies of CODM were cloned from genomic DNA of a BC₂F₂ high codeine individual genotyped as homozygous for R158K and E193K mutations/polymorphisms (see Example 3). At least seven different CODM genomic gene sequences were obtained (SEQ ID NO 1-7, Table 1) giving rise to seven deduced transcribed and four deduced amino acid sequences. Thus there are at least four copies of CODM in the genome of the diploid opium poppy (assuming that the identified sequences represent the maximum number of theoretically possible allelic variants) or up to seven copies (assuming no allelic variation among the CODM sequences identified). The R158K and E193K mutations/polymorphisms co-segregated together through subsequent generations when generating the BC₂F₂ population indicating close linkage of at least three copies of CODM (SEQ ID NO 2, 3 and 6; three copies since R158K occurs in two different CODM copies).

### Example 2

### A knock out mutation in one copy of the CODM genes leads to a limited increase in codeine

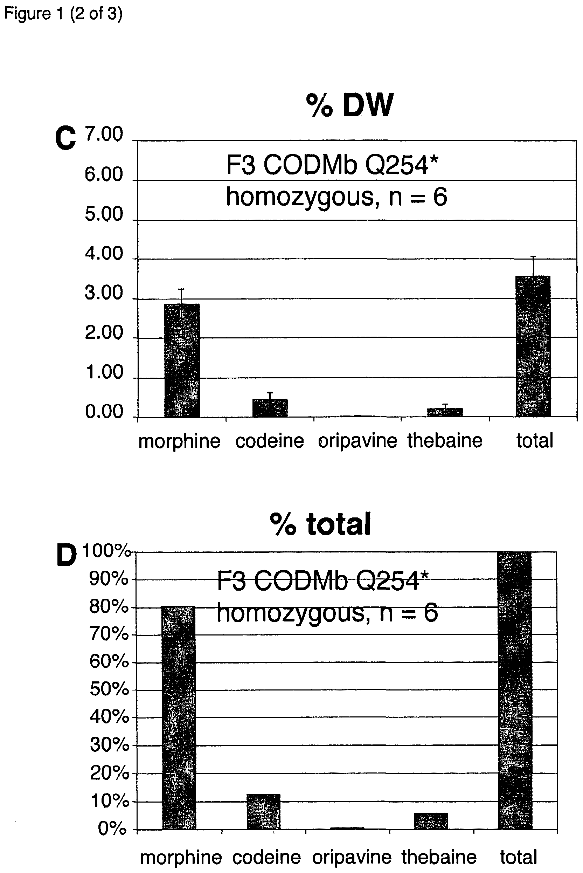

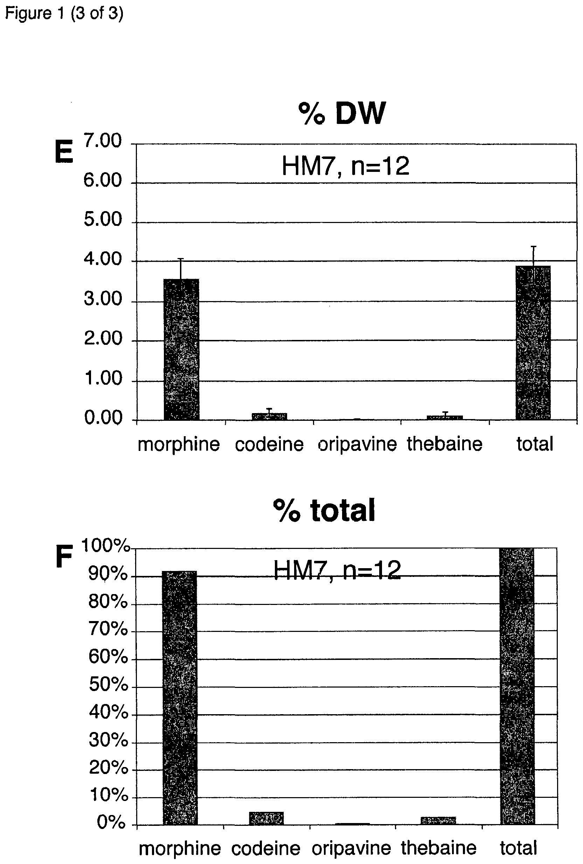

Reverse genetic screening of the CODM genes yielded a pre-mature stop codon mutation in CODM (Q254*) which likely renders the resulting truncated protein non-functional. CODM Q254* plants were crossed to morphine cultivar HM6 and the effect of the mutation analysed in F3 material grown in the field in Tasmania. Homozygous F3 material showed a 2-fold increase of codeine (0.46 ± 0.16%) compared to HM6 (0.18 ± 0.10% DW, Figure 1C and E). Likewise, the relative amount of codeine of total major alkaloids (morphine, codeine, oripavine and thebaine) is with 12% more than twice as high than that in HM6 (5%, Figure 1 D and F). The limited uplift in codeine by this putative CODM knock out mutation is explained by having multiple functionally redundant copies CODM (Example 1).

### Example 3

### Amino acid substitution polymorphisms/mutations in three copies of CODM, are associated with elevated codeine and decreased oripavine levels indicating decreased CODM activity

Field-based forward screening of EMS mutagenised HM2 material led to the discovery of a M2 mutant plant with higher codeine levels than non-mutagenised HM2 material. The M2 plant originated from M2 seed family S-103234 (Table 2). The increased codeine content was confirmed in the self-pollinated M3 to M7 generations grown in consecutive growing seasons in the field in Tasmania showing that the high codeine trait is heritable. Sequencing of the CODM genes from this material revealed mutations/polymorphism in three copies of CODM leading to amino acid substitutions, R158K (in SEQ ID NO 3 and 6) and E193K (in SEQ ID NO2). KASP marker assays were designed to track the mutation in marker-assisted crosses. Using these markers the M7 generation (MAS1983 C) was crossed to the recurrent parent high morphine cultivar HM6. The R158K and E193K mutations/polymorphisms were segregating together indicating close linkage between the respective CODM copies they reside in.

F3 plants homozygous for both mutations were grown in the field in Tasmania in the 2014/15 season alongside the recurrent HM6 parent (Figure 1A and D). Analysis of this F3 material showed a 5-fold increase of codeine (1.10 ± 0.27% DW) compared to the recurrent parent HM6 (0.18 ± 0.10% DW, Figure 1A and E). The relative amount of codeine reached 18% compared to 5% in HM6 (Figure 1B and F).

Following further crosses to the recurrent parent HM6, the BC₂F₁ generation was self-pollinated and the segregating BC₂F₂ grown to maturity in the glasshouse to assess the impact of the mutations on codeine levels. The BC₂F₂ plants homozygous for the mutations contained on average twice as much codeine as those plants homozygous for the wild type alleles (0.80 ± 0.37 % DW versus 0.37 ± 0.21 % DW; Figure2A). Capsules from heterozygous plants contained intermediate levels. Consistent with an impact on CODM activity in the mutant plants, oripavine levels were about five times lower in homozygous mutant BC₂F₂ material compared to material homozygous for the wild type alleles (Figure 2A).

The relative amount of codeine was more than twice as high in capsules of homozygous mutant plants than in those from plants homozygous for the wild type alleles (19.4% versus 8.9%, Figure 2B).

The co-segregation of the CODM mutations/polymorphisms R158K and E193K in their respective CODM copies with higher codeine content shows that these mutations are linked to - if not causative for - the high codeine trait.

### Example 4

### The complete absence of CODM activity in a fast neutron mutagenised plant is caused by a deletion of all CODM genes leading to a complete loss of morphine and oripavine and gain of codeine

Glasshouse-based forward screening of fast neutron mutagenised material of the high morphine and noscapine cultivar HN2 yielded a M2 mutant plant (R80624_G07) that completely lacked morphine and oripavine but had gained high levels of codeine indicating a complete loss of CODM activity. The plant had retained its capacity to synthesise noscapine. The M2 mutant plant was self-pollinated and the heritability of the codeine and noscapine phenotype was confirmed in M3 capsules (Figure 3). M3 plants contained on average 1.9 % dry weight each of codeine and noscapine which accounted in each case for nearly 44% of total alkaloids. The M3 generation, too, was self-pollinated. M4 progeny was grown and DNA isolated. Using a number of primers in various combinations failed to amplify any of the CODM genes in the M4 mutant plants whereas amplification was successful on DNA from non-fast neutron mutagenised morphine synthesising control plants (Figure 4). The complete lack of amplification of CODM genes suggests that the complete loss of CODM activity observed in capsule material derived from high codeine mutant R80624_G07 is a consequence of a deletion of all the CODM genes from its genome. This in turn indicates that all copies of CODM are closely linked.

### Example 5

### The CODM genes are clustered in the opium poppy genome

BAC sequencing and assembly revealed that the CODM genes occur as a cluster of at least three CODM genes within the opium poppy genome (Figure 5). This finding is consistent with the fact that all CODM genes are deleted in a fast neutron mutagenised plant that accumulates high levels of codeine but lacks morphine (Figure 4 and example 4). Fast neutron mutagenesis has been shown to be capable of inducing deletions ranging from several hundred to tens of thousands of base pairs (O'Rourke et al. (2013) Frontiers in Plant Science 4: Article 210). The lack of amplification of CODM fragments in the fast neutron mutagenised high codeine plant (Figure 4) indicates that the entire CODM gene cluster has been deleted.

## Claims

1. A Papaver plant species wherein the plant is modified by deletion for all or part of three linked genes encoding codeine 3-O-demethylase comprising the nucleotide sequence set forth in SEQ ID NO: 7, or a nucleotide sequence that is 99% identical to SEQ ID NO: 7, **characterised in that** the activity of said codeine 3-O-demethylase is undetectable and wherein the plant has a codeine content of at least 45% of the total alkaloid content of latex or dried straw extracted from the modified plant.

2. The *Papaver* plant according to claim 1 wherein the plant is modified by deletion of a nucleic acid molecule comprising or consisting of a nucleotide sequence as set forth in SEQ ID NO: 44.

3. The *Papaver* plant according to claim 1 or 2 wherein the codeine content is at least 50%, 60%, 70%, 80%, 90%, 95%, 96%, 97% or 98% of the total alkaloid content of latex or dried straw extracted from said modified plant.

4. The *Papaver* plant according to any one of claims 1 to 3 wherein the codeine content is at least 0.8% of the dried straw of said modified plant.

5. The *Papaver* plant according to claim 3 or 4 wherein the codeine content is between 0.8% and 10.0% of the dried straw.

6. The *Papaver* plant according to claim 5 wherein the codeine content is between 0.8% and 3.0% of the dried straw.

7. The *Papaver* plant according to claim 6 wherein the codeine content is between 3.0% and 10.0% of the dried straw

8. The *Papaver* plant according to claim 6 wherein the codeine content is between 1.0% and 1.5% of the dried straw.

9. The *Papaver* plant according to claim 1 or 2 wherein said plant has a codeine content that is at least 55% of the total alkaloid content of latex or dried straw extracted from said modified plant or has a codeine content that is at least 0.8% of the dried straw.

10. The *Papaver* plant according to any one of claims 1 to 9 wherein said plant has undetectable levels of morphine.

11. The *Papaver* plant according to any one of claims 1 to 9 wherein said plant lacks morphine and oripavine.

12. The *Papaver* plant according to any one of claims 1 to 11 wherein said plant is modified by Fast Neutron Mutagenesis,

13. The Papaverplant according to any one of claims 1 to 12 wherein said plant is *Papaver somniferum.*

14. A process for the extraction of codeine and/or related codeine alkaloids from a *Papaver* plant comprising the steps:
i) harvesting a plant or plant material prepared from a plant according to any one of claims 1 to 13;
ii) forming a reaction mixture of particulate plant material;
iii) solvent extraction of the reaction mixture to provide an alkaloid enriched fraction; and
iv) concentrating said alkaloid enriched fraction to provide a codeine enriched fraction.

## Patentansprüche

1. Papaver-Pflanzenspezies, wobei die Pflanze durch Deletion für alle oder einen Teil von drei verknüpften Genen modifiziert ist, die Codein 3-0-demethylase codieren, umfassend die in SEQ ID NR.: 7 dargelegte Nukleotidsequenz oder eine Nukleotidsequenz, die zu 99 % identisch mit SEQ ID NR.: 7 ist, **dadurch gekennzeichnet, dass** die Aktivität der Codein 3-0-demethylase nicht nachweisbar ist, und wobei die Pflanze einen Codeingehalt von wenigstens 45 % des Gesamtalkaloidgehalts von Latex oder getrocknetem Stroh aufweist, das aus der modifizierten Pflanze extrahiert wurde.

2. *Papaver-Pflanze* nach Anspruch 1, wobei die Pflanze durch Deletion eines Nukleinsäuremoleküls modifiziert ist, umfassend oder bestehend aus eine(r) Nukleotidsequenz, wie in SEQ ID NR.: 44 dargelegt.

3. *Papaver*-Pflanze nach Anspruch 1 oder 2, wobei der Codeingehalt wenigstens 50 %, 60 %, 70 %, 80 %, 90 %, 95 %, 96 %, 97 % oder 98 % des Gesamtalkaloidgehalts von Latex oder getrocknetem Stroh beträgt, das aus der modifizierten Pflanze extrahiert wurde.

4. *Papaver*-Pflanze nach einem der Ansprüche 1 bis 3, wobei der Codeingehalt wenigstens 0,8 % des getrockneten Strohs der modifizierten Pflanze beträgt.

5. *Papaver*-Pflanze nach Anspruch 3 oder 4, wobei der Codeingehalt zwischen 0,8 % und 10,0 % des getrockneten Strohs beträgt.

6. *Papaver*-Pflanze nach Anspruch 5, wobei der Codeingehalt zwischen 0,8 % und 3,0 % des getrockneten Strohs beträgt.

7. *Papaver-Pflanze* nach Anspruch 6, wobei der Codeingehalt zwischen 3,0 % und 10,0 % des getrockneten Strohs beträgt.

8. *Papaver-Pflanze* nach Anspruch 6, wobei der Codeingehalt zwischen 1,0 % und 1,5 % des getrockneten Strohs beträgt.

9. *Papaver-Pflanze* nach Anspruch 1 oder 2, wobei die Pflanze einen Codeingehalt aufweist, der wenigstens 55 % des Gesamtalkaloidgehalts von Latex oder getrocknetem Stroh beträgt, das aus der modifizierten Pflanze extrahiert wurde, oder einen Codeingehalt aufweist, der wenigstens 0,8 % des getrockneten Strohs beträgt.

10. *Papaver*-Pflanze nach einem der Ansprüche 1 bis 9, wobei die Pflanze nicht nachweisbare Morphinspiegel aufweist.

11. *Papaver*-Pflanze nach einem der Ansprüche 1 bis 9, wobei der Pflanze Morphin und Oripavin fehlt.

12. *Papaver*-Pflanze nach einem der Ansprüche 1 bis 11, wobei die Pflanze durch schnelle Neutronen-Mutagenese modifiziert ist,

13. Papaver-Pflanze nach einem der Ansprüche 1 bis 12, wobei die Pflanze Papaver *somniferum* ist.

14. Verfahren zur Extraktion von Codein und/oder verwandten Codeinalkaloiden aus einer *Papaver*-Pflanze, umfassend die Schritte:
i) Ernten einer Pflanze oder von Pflanzenmaterial hergestellt aus einer Pflanze nach einem der Ansprüche 1 bis 13;
ii) Bilden eines Reaktionsgemisches aus partikulärem Pflanzenmaterial;
iii) Lösungsmittelextraktion des Reaktionsgemisches zum Bereitstellen einer alkaloidangereicherten Fraktion; und
iv) Konzentrieren der alkaloidangereicherten Fraktion zum Bereitstellen einer codeinangereicherten Fraktion.

## Revendications

1. Espèce de plante Papaver dans laquelle la plante est modifiée par délétion pour tout ou partie de trois gènes liés codant pour la codéine 3-O-déméthylase comprenant la séquence nucléotidique présentée dans SEQ ID N° : 7, ou une séquence nucléotidique qui est identique à 99 % à SEQ ID N° : 7, **caractérisée en ce que** l'activité de ladite codéine 3-O-déméthylase est indétectable et dans laquelle la plante a une teneur en codéine d'au moins 45 % de la teneur totale en alcaloïdes de latex ou de paille séchée extraits à partir de la plante modifiée.

2. Plante *Papaver* selon la revendication 1, dans laquelle la plante est modifiée par délétion d'une molécule d'acide nucléique comprenant ou constituée d'une séquence nucléotidique telle que présentée dans SEQ ID N° : 44.

3. Plante *Papaver* selon la revendication 1 ou 2, dans laquelle la teneur en codéine est d'au moins 50 %, 60 %, 70 %, 80 %, 90 %, 95 %, 96 %, 97 % ou 98 % de la teneur totale en alcaloïdes de latex ou de paille séchée extraits à partir de ladite plante modifiée.

4. Plante *Papaver* selon l'une quelconque des revendications 1 à 3, dans laquelle la teneur en codéine est d'au moins 0,8 % de la paille séchée de ladite plante modifiée.

5. Plante *Papaver* selon la revendication 3 ou 4, dans laquelle la teneur en codéine est entre 0,8 % et 10,0 % de la paille séchée.

6. Plante *Papaver* selon la revendication 5, dans laquelle la teneur en codéine est entre 0,8 % et 3,0 % de la paille séchée.

7. Plante *Papaver* selon la revendication 6, dans laquelle la teneur en codéine est entre 3,0 % et 10,0 % de la paille séchée.

8. Plante *Papaver* selon la revendication 6, dans laquelle la teneur en codéine est entre 1,0 % et 1,5 % de la paille séchée.

9. Plante *Papaver* selon la revendication 1 ou 2, dans laquelle ladite plante a une teneur en codéine qui est d'au moins 55 % de la teneur totale en alcaloïdes de latex ou de paille séchée extraits à partir de ladite plante modifiée ou a une teneur en codéine qui est d'au moins 0,8 % de la paille séchée.

10. Plante *Papaver* selon l'une quelconque des revendications 1 à 9, dans laquelle ladite plante a des niveaux indétectables de morphine.

11. Plante *Papaver* selon l'une quelconque des revendications 1 à 9, dans laquelle ladite plante est dépourvue de morphine et d'oripavine.

12. Plante *Papaver* selon l'une quelconque des revendications 1 à 11, dans laquelle ladite plante est modifiée par mutagenèse par neutrons rapides,

13. Plante *Papaver* selon l'une quelconque des revendications 1 à 12, dans laquelle ladite plante est Papaver *somniferum.*

14. Procédé pour l'extraction de codéine et/ou d'alcaloïdes de codéine apparentés à partir d'une plante *Papaver* comprenant les étapes :
i) de récolte d'une plante ou d'une matière végétale préparée à partir d'une plante selon l'une quelconque des revendications 1 à 13 ;
ii) de formation d'un mélange réactionnel de matière végétale particulaire ;
iii) d'extraction par solvant du mélange réactionnel pour obtenir une fraction enrichie en alcaloïdes ; et
iv) de concentration de ladite fraction enrichie en alcaloïdes pour obtenir une fraction enrichie en codéine.
